# EUROPEAN PATENT SPECIFICATION

(11) **EP 1 221 969 B1**
(45) Date of publication and mention of the grant of the patent: **14.04.2010**
(21) Application number: 00969488.6
(22) Date of filing: 11.10.2000
(51) Int. Cl.: A61K 39/00, A61K 35/14, C12N 5/071, A61P 35/00, A61P 31/00, A61P 37/00

(54) **ACTIVATION OF ANTIGEN-SPECIFIC T CELLS BY VIRUS/ANTIGEN-TREATED DENDRITIC CELLS**
AKTIVIERUNG ANTIGEN-SPEZIFISCHER T-ZELLEN MIT VIRUS/ANTIGEN-BEHANDELTEN DENDRITISCHEN ZELLEN
ACTIVATION DE CELLULES T A SPECIFICITE ANTIGENIQUE PAR DES CELLULES DENDRITIQUES TRAITEES AVEC UN VIRUS/ANTIGENE

(30) Priority: 13.10.1999 EP 99119980
(43) Date of publication of application: 17.07.2002
(73) Proprietor: Ahlert, Thorsten Dr., Scarborough, YO12 6EH North Yorkshire (GB)
(72) Inventor: Ahlert, Thorsten Dr., Scarborough, YO12 6EH North Yorkshire (GB)
(74) Representative: Müller-Boré & Partner Patentanwälte
(86) International application number: PCT/EP2000/010019
(87) International publication number: WO 2001/026680

(56) References cited:
- EP-A- 0 331 102
- WO-A-94/21798
- C. ERTEL ET AL.: "VIRAL HEMAGGLUTININ AUGMENTS PEPTIDE-SPECIFIC CYTOTOXIC T CELL RESPONSES." EUROPEAN JOURNAL OF IMMUNOLOGY, vol. 23, 1993, pages 2592-2596, XP002911370 WEINHEIM, DE
- T. TÜTING ET AL.: "AUTOLOGOUS HUMAN MONOCYTE-DERIVED DENDRITIC CELLS GENETICALLY MODIFIED TO EXPRESS MELANOMA ANTIGENS ELICIT PRIMARY CYTOTOXIC T CELL RESPONSES IN VITRO: ENHANCEMENT BY COTRANSFECTION OF GENES ENCODING THE Th1-BIASING CYTOKINES IL-12 AND IFN-ALPHA" JOURNAL OF IMMUNOLOGY, vol. 160, 1998, pages 1139-1147, XP002132188 BALTIMORE US
- DATABASE BIOSIS [Online] BIOSCIENCES INFORMATION SERVICE, PHILADELPHIA, PA, US; 1976 MARTZ E: "SIZES OF ISOTOPICALLY LABELED MOLECULES RELEASED DURING LYSIS OF TUMOR CELLS LABELED WITH CHROMIUM-51 AND CARBON-14 NICOTINAMIDE" Database accession no. PREV197865047395 XP002132189 & CELLULAR IMMUNOLOGY 1976 * EN *, vol. 26, no. 2, 1976, pages 313-321, ISSN: 0008-8749
- V. SCHIRRMACHER ET AL.: "HUMAN TUMOR CELL MODIFICATION BY VIRUS INFECTION: AN EFFICIENT AND SAFE WAY TO PRODUCE CANCER VACCINE WITH PLEIOTROPIC IMMUNE STIMULATORY PROPERTIES WHEN USING NEWCASTLE DISEASE VIRUS." GENE THERAPY, vol. 6, no. 1, January 1999 (1999-01), pages 63-73, XP000877315 BASINGSTOKE, GB cited in the application

## Description

The present invention relates to a method for the preparation of a composition containing T cell activating agent containing dendritic cells (DC) treated with virus-treated antigen which may be used as a vaccine to stimulate an immune response in a patient.

The immune system of cancer patients but also of patients suffering from chronic infectious diseases, autoimmune diseases, renal failure with the need of dialysis, or inherited immune dysfunctions works inefficient and needs external help. The causes for this immune deficiency may be the diseases themselves or externally induced defects including immunosuppression by conventional cancer therapies. Furthermore, the recognition of diseases like cancer or infections by the immune system may face obstacles such as weak or inefficient presentation of disease-specific antigen.

Thus, reconstitution of immunocompetence for specific antigens is an object of intense research. Cell therapy by transfer of *in vitro*-activated allogeneic or autologous antigen-specific T cells or *in vivo* induction of such cells by vaccination procedures are current approaches aiming to solve the above-mentioned problems of immune deficiency.

T cells are lymphocytes which are able to provide specific and nonspecific immunologic help for several immune mechanisms and which may also directly attack and eradicate foreign or disease-associated cellular antigens. They are able to develop and/or transfer immunologic memory over months and years towards such antigens and thus, are important mediators of long-term immunologic protection.

The first approaches developed to confer an improved competence to a patient suffering from immune deficiency were adoptive cell transfer therapies. These therapies were mainly used for the treatment of cancer and employed in the first instance the so-called lymphokine-activated killer cells (LAK) and later lymphokine-activated tumor-infiltrating lymphocytes (TIL) for autologous transfer. The term "autologous" means that the cells which were transferred originated from the patient him- or herself. The killer cells were generated from peripheral blood or from tumor tissue which was freshly obtained by operation. The obtained killer cells were cultivated and activated mainly in medium containing interleukin 2 (IL-2), a T cell growth factor [refs. 1.,2.]. Other attempts of adoptive autologous and allogeneic cell transfer include stimulation of T cells with tumor cells and/or antibodies or cytokines *in vitro* or *in vivo* before they are adoptively transferred to the recipient [refs. 3. to 7.]. The term "allogeneic" means that the transferred cells originated from a nonidentical individual.

The use of dendritic cells (DC) for the *in vivo* or *in vitro* activation of antigen (peptide)-specific T cells has been established in very recent years [refs. 8. to 13.]. DCs are "professional" antigen-presenting cells which can be more powerful antigen-specific activators of T cells than the antigen-bearing cells themselves. DC can be pulsed or loaded with antigens such as peptides or cell lysates, for example antigens derived from tumor cells. The DCs process the antigenic material and integrate the products into MHC class I and/or class II complexes which are able to present them to T cells. For a full activation of killer T cells, a presentation of processed material in both class I and II MHC complexes is necessary, although the killer cells themselves are only able to recognise a presentation via MHC class I. MHC class II is necessary for the additional activation of helper T cells. This finding has led to the addition of substitutes like Keyhole-limpet haemocyanin (KLH) which represent MHC class II integrateable helper antigens. Furthermore, such a substitute would be able to represent a neoantigen and thus, can serve as a tracer molecule [ref. 8.].

Newcastle Disease Virus (NDV) is an avian paramyxovirus which has been used for a long time in cancer therapy. This virus can be used directly for infection and lysis of cancer cells *in vitro* or *in vivo,* and it can be used for modification of tumor cells in vaccines in order to give rise to an improved adhesion of stimulatory cells to their target cells. NDV may also induce a broad range of co-stimulatory signals for T cell activation when it is used for modification in cellular vaccines [refs. 14. to 22.].

However, the above-mentioned approaches display several problems and disadvantages.

In case of the use of cytokines for the activation and expansion of immune cells *in vitro,* the methods which have been used previously have not shown acceptable clinical risk-benefit relations. This is mostly due to an unspecific activation of the whole immune system and a dependency of the transferred cells on *in vivo* cytokine substitution after the application to the patient. The resulting treatment of patients with high-dose cytokines is accompanied by significant side effects which have led to an abandonment of this approach. More sophisticated methods using an antigen-specific component or a T cell receptor trigger for *in vitro* activation of T cells in addition to low-dose cytokines yielded cells which showed only a limited activity. Usually, the activity of the cells is readily suppressed after transfer into the patient, or the immune cells do not find (i.e. do not migrate to) the targeted cells *in vivo.* Therefore, the immunologic memory generated in this way is only short-lived and, moreover, leads to early disease progression after occasional therapeutic success.

Until now, DCs have been used clinically for *in vivo* induction of antigen-specific immune responses. However, obstacles which prevented a successful therapy using DCs have been the generation and/or isolation of a sufficient amount or functionally active DCs for therapeutic application. The strategies which have been developed until now, have rarely considered the possibility of a tolerance induction by insufficiently pulsed or insufficiently differentiated DCs. Furthermore, the *in vivo* generation of T cell responses with DCs can be difficult in patients with a deficient immune system.

The *in vivo* oncolysis using NDV has turned out to be difficult because of an efficient inactivation of the virus by the patient's immune system and because of virus-resistant tumor cells in heterogeneous tumors. Virus-modified tumor cell vaccines which have been used until now for *in vivo* activation of antigen-specific T cells show only limited effects which are only observed in early cancer stages. Approaches using NDV face further obstacles such as immune suppressed or immune deficient patients, insufficiently active T cells because of an antigen overload in the vaccinated patient or because of inhibitory factors which are produced by the target cells for T cells. Furthermore, suboptimal antigen presentation on antigen-varying target (i.e., in this case, tumor) cells and on *in vivo* preexisting antigen-presenting cells can be the reason for the failure of T cell activation.

A further problem of virus-modified cell vaccines which have been developed until now is that they need a significant number of viable antigen-bearing cells in order to reach a sufficient efficiency. This has limited the use of virus-modified cell vaccines in clinical applications so far because in clinical situations a comparably large amount of raw material (mostly surgically resectable tumor material) is available which contains considerable amounts of dead cells.

Moreover, the necessity of malignant viable cells in virus-modified vaccines results in the need of irradiation of the cells for their inactivation before *in vivo* use. This inactivation method is effective, however, it is difficult to apply with respect to the controlled pharmaceutical production process [refs. 20., 23. to 25.].

WO 94/21798 describes *inter alia* the medical use of DNA encoding of the haemagglutinin-neuramidase (HN) of a paramyxovirus or the protein thereof, in each case free of its native paramyxovirus, for the purpose of increasing the antigen-specific T cell responses in humans. Tumor cells are inactivated, infected with HN DNA or loaded with HN protein and then administered back to the patient. In a second possibility, macrophages or other antigen-presenting cells are isolated from a patient or are grown in culture and matched with the patients MHC, incubated with a T-cell epitope-bearing peptide or other suitable antigen and infected with the HN DNA or loaded with HN protein, whereby in the patient the *in vivo* stimulation of cytotoxic T-cells of high specificity for cytolysis of diseased cells is increased by the HN protein-providing component.

Therefore, the technical problem underlying the present invention is to improve the *in vivo* and *in vitro* induction of highly active antigen-specific immune stimulators, the effect of which is especially mediated by T cells and T memory cells during therapeutic clinical use. This improvement includes the reduction of the possibility of tolerance induction by T cell activation efforts and, thus, increasing the safety of the procedure.

The solution of the above technical problem is achieved by providing the embodiments as characterized in the claims.

In particular, the present invention relates to a method for the preparation of a composition containing activated T cells which are capable of performing and stimulating a specific immune response in a patient, comprising the steps of
(i) preparing T cells from the patient or a relative thereof;
(ii) activating dendritic cells by a method comprising the steps of
   (a) preparing as an antigen a tumor cell infected with newcastle disease virus (NDV) *in vitro,*
   (b) preparing monocytes from the patient or a relative thereof,
   (c) developing dendritic cells from the monocytes by incubation *in vitro,*
   (d) coincubating the obtained dendritic cells with the antigen obtained in step (a) *in vitro,*
   wherein the virus is capable of improving the adhesion of the antigen to and the presentation of the antigen by the dendritic cells and of modulating the activation, maturation, stability and cosignalling of the dendritic cells;
(iii) preparing a T cell activating agent comprising the dendritic cells of step (ii); and
(iv) activating the T cells of step (i) by treatment with the T cell activating agent of step (iii) *in vitro,*
wherein steps (i), (ii) (a), and (ii) (b) do not involve any surgical treatment of a human's or animal's body and wherein the relative of the patient is a person or animal being related by blood and/or genetically via HLA-type with the patient.

Further, the present invention also relates to a method for the preparation of a T cell activating agent containing activated dendritic cells for the activation of T cells which perform and stimulate a specific immune response in a patient, wherein the dendritic cells are activated by the method comprising the steps of
(a) preparing as an antigen a tumor cell infected with newcastle disease virus (NDV) *in vitro,*
(b) preparing monocytes from the patient or a relative thereof,
(c) developing dendritic cells from the monocytes by incubation *in vitro,*
(d) coincubating the obtained dendritic cells with the antigen obtained in step (a) *in vitro,*
wherein the virus is capable of improving the adhesion of the antigen to and the presentation of the antigen by the dendritic cells and of modulating the activation, maturation, stability and cosignalling of the dendritic cells, wherein steps (a) and (b) do not involve any surgical treatment of a human's or animal's body, and wherein the relative of the patient is a person or animal being related by blood and/or genetically via HLA-type with the patient.

The term "T cell activating agent" as used herein means a composition or formulation containing activated dendritic cells which are capable of stimulating an immune response in a patient against antigens. The activated dendritic cells of the above-defined T cell activating agent are capable of activating T cells *in vivo* as well as *in vitro.*

Therefore, the above-defined composition in which the T cell activating agent as characterized above may be used for T cell activation *in vitro.* The T cell activating agent itself may be used as a vaccine for T cell activation *in vivo*.

Thus, the present invention provides novel methods for the improvement of both *in vivo* and *in vitro* induction of highly active, antigen-specific immune stimulators which may be used in the two following different therapeutic regimens:
(1) The composition obtained by the method of the present invention is particularly useful in a method of cellular therapy, wherein the activated T cells are administered to the patient. Such a method is also referred to as adoptive or passive immunotherapy (ADI).
(2) The T cell activating agent obtained according to the present invention provides a vaccine, e.g. a tumor vaccine, for immunizing a patient with an antigen such as a tumor antigen in a highly immuogenic form. Such a therapeutic regimen is also referred to as active specific immunotherapy (ASI).

For stimulating T cells *in vivo,* the activated dendritic cells may be administered, e.g. intracutaneously, subcutaneously or intralymphatically, to the patient, and patient's T cells migrate to the administration locus where they are activated by the dendritic cells. The T cell activating agent used for the activation of T cells in the composition obtained according to the present invention may also contain substances which are prepared using recombinant DNA technology. Preferably, the T cell activating agent used for T cell activation in the composition obtained according to the present invention contains one or more other T cell activating agents which act additively or synergistically with the dendritic cells.

The term "antigen" comprises any structure which is capable of inducing an immune response in an organism either by itself or when coupled to a suitable carrier molecule or cell. Therefore, antigens according to the present invention are tumor cells as well as the parts thereof such as polypeptides, oligopeptides derived therefrom, lipids such as glycolipids, polysaccharides and nucleic acids. According to a preferred embodiment of the above-definedcomposition, the antigen is prepared from the patient, however, as defined above, the antigen may as well be prepared from other organisms or may be synthetic or biosynthetic.

Preferably, the antigen which is virus-treated in step (ii) (a) such as virus-treated living tumor cells may be inactivated without the use of irradiation prior to the coincubation with the dendritic cells in step (d) of the above-defined method. Preferred methods for inactivation and lysis of living cells such as living tumor cells include, for example, freeze-thawing and ultrasonification. The use of methods apart from irradiation poses less problems to the pharmaceutical production process.

The use of bone marrow in addition or instead of blood as the source for T cells which are to be activated leads to an increase of the yield of highly active antigen-specific T cells, for example T memory cells, for the stimulation by coincubation with NDV-modified DCs.

Preferably, the antigen such as a cell, e.g. a tumor cell, may be further purified during the preparation from the patient, for example by immunobead techniques. These techniques comprise the use of small magnetic metal beads (e.g. from Dynal or Milteny) which are coupled to antibodies directed against contaminating components such as cells or other agents. After having bound to the antibody-coupled beads during an incubation step, the contaminations are removed from the T cell activating agent, e.g. a suspension of the activated dendritic cells, by applying a magnetic field which draws the beads out of the suspension. Further, the cells may be cryoconservated after their preparation, e.g. from the patient, in step (a) above and may be thawed before or after treatment with the virus in step (a) and/or coincubation with the dendritic cells in step (d) above.

Since dendrtitic cells are capable of processing antigens derived from genetic material, it is also possible to use genetic material, i.e. a nucleic acid such as DNA or RNA (preferably mRNA), encoding the immunological signals said virus-treated/modified antigen provides, for activating (pulsing) dendritic cells instead of or in addition to the antigen itself in step (d) of the method for DC activation as defined above. For example, the dendritic cells may be treated with the corresponding nucleic acid(s) by transfection (e.g. using Ca-phosphate, lipofection or electroporation methods). Preferred sources of the nucleic acid(s) are virus-infected antigen presenting cells. These cells process not only gene products for antigen expression, but also products, e.g. a cocktail of cytokines, heat shock proteins etc., induced by virus infection serving as immunological signals. For example, the mRNA coding for such products may be transcribed into DNA and thereafter this genetic material may be amplified by the use of PCR. Thus, a constant source of virus-treated/modified antigen for continued treatment of large numbers of patients can be provided which is pharmaceutically easy to handle.

Preferably, the developing dendritic cells are also coincubated with the virus during the step of incubation *in vitro* (c).

According to further preferred embodiments of the method for obtaining the above-defined composition and the T cell activating agent, the virus used is selected from the group consisting of paramyxoviruses such as newcastle disease virus (NDV) or mumps virus, vaccinia virus, myxovirus, herpesvirus, AIDS virus, human papillomavirus (HPV) and mouse mammary tumor virus (MMTV).

The T cell activating agent used for T cell activation in the composition obtained according to the present invention comprises dendritic cells which are activated with a virus and an antigen which is preferably prepared from a patient having a significantly impaired immune system. Preferably, this impairment of a patient's immune system may be caused by chronic disorders such as cancer, infections, renal failure which has to be treated by dialysis, autoimmune diseases and/or inherited immune dysfunctions.

The term "patient" as used herein comprises humans as well as animals. The preferred patient is a human. The "relative" of the patient is a person or animal, respectively, being related by blood and/or genetically via HLA-type with the patient, i.e. the human or animal.

In the above-defined composition the T cells are activated by the method comprising the steps of
(i) preparing T cells from the patient or relative thereof, and
(ii) treating the T cells with the T cell activating agent as defined above *in vitro.*

Preferably, the treatment of T cells with the T cell activating agent according to the present invention in step (ii) above is carried out by coincubation in a low- or medium-dose cytokine-containing medium for a short time. More preferably, the culture medium contains not more than 6000 U/ml of cytokines, for example IL-2, and the cultivation in the low-dose cytokine-containing medium is not longer than seven days.

According to preferred embodiments of the present invention at least part of the monocytes prepared in step (b) of the above-defined T cell activating agentand at least part of the T cells prepared in step (i) of the above-defined composition may be derived from the patient's or relative's bone marrow or blood. Therefore, in contrast to prior art cell therapy vaccines, bone marrow may be used in the above-defined T cell activating agent as a very efficient source of monocytes from which dendritic cells are developed and, furthermore, in the above-defined composition as a very efficient source of (memory) T cells which are obtained for *in vitro* activation with virus-treated DCs in addition to monocytes or T cells, respectively, from peripheral blood.

In addition to the above-mentioned advantages of the T cell activating agent and the composition obtained according to the present invention, they show several further advantages due to the novel approach for the activation of T cells.

1,5x10⁶ NDV-modified DCs and an equivalent of 1,5x10⁶ target cells (for example tumor cells) when used as the antigen are needed for human *in vivo* vaccination or for a reasonably effective *in vitro* stimulation of T cells derived, for example, from humans. Taking these considerations into account the method for T cell activation as described above provides the possibility to use target cells as antigens which may be either dead or alive, since the uptake and processing of the material by the DCs leads to an antigen presentation to living TCs in the end. In contrast, in conventional virus-modified tumor cell vaccines at least 1,5x10⁶ target cells must be alive in order to lead to an efficient T cell activation and no more than 66% of dead cells should contaminate the living target cells [refs. 20., 24. to 26.]. However, an average of 50% of the target cells are dead in, for example, fresh tumor cells suspensions after cryoconservation which is mostly necessary for the storage of the raw material. Therefore, the use of NDV-modified DCs instead of original antigen-bearing living target cells reduces about 50% of the amount of raw material needed, since dead target cells as well as living target cells can be used as the antigen (the living target cells are preferably disintegrated by shock freezing or by the infection with the virus).

Furthermore, the use of a virus, for example NDV, in order to increase the DC number and to enhance their function facilitates and increases the generation of efficient DCs which can be used for *in vitro* or *in vivo* activation of (memory) T cells.

The use of a virus, for example a paramyxovirus such as NDV, which is capable of improving the adhesion of the antigen to the dendritic cells and which is capable of stimulating the activation of the dendritic cells as described above further reduces the probability of an induction of tolerance by an inefficient number and/or function of DCs in the patient. This advantage and the fact that the use of a virus as described above for increasing the number of DCs as well as their function improves and increases the generation of efficient DCs represent further surprising properties of the T cell activating agent according to the present invention which can not be predicted from known properties of viruses such as NDV in tumor cell modification. Generally, DCs *per se* should be able to perform an optimal antigen presentation function and a costimulatory signalling for T cell activation. Therefore, in theory, there is no obvious need for the effects of a virus like NDV on DCs. However, while a virus such as NDV in fact induces a secretion of costimulatory cytokines and provides adhesion molecules for longer T cell-target cell interaction for the preparation of cellular vaccines such as the above described tumor cell vaccines, it improves the maturation and/or differentiation and/or antigen presentation, respectively, of DCs. Furthermore, the virus such as NDV may also modify in addition or instead of inducing a costimulatory signalling in DCs in order to generate an improved T cell activation.

Also, according to preferred embodiments of the composition and the T cell activating agent obtained according to the present invention, the virus such as NDV is capable of inducing at least in part fusions between the antigen and the dendritic cells in step (d) of the activation of the dendritic cells. The fusion may be mediated via the virus' fusion protein leading to hybrids between the dendritic cells and virus-treated antigen such as a cell. Such hybrids further improve the antigen-presenting capability and T cell activating activities of dendritic cells. The antigen is preferably a cell such as a tumor cell derived from a patient or a cell derived from a tumor cell line which confers the hybrid with multiple tumor-associated antigens.

These effects on DCs were not expected from the known properties of viruses such as NDV on tumor cells and tumor cell vaccines. On the contrary, one would have expected from prior art studies that viruses suppress DCs. For example, Jenne et al. [ref. 27.] found a suppression of T cell stimulation properties by viruses and Raftery et al. [ref. 28.] found changes in DC function which were believed to contribute to human cytomegalovirus-associated immunosuppression after infection of DC with human cytomegalovirus.

Furthermore, the T cells which are contained in the composition obtained according to the present invention and which are activated by the above-described method using the above-defined T cell activating agent *in vitro,* exhibit a high efficiency and reduced dependency on *in vivo* application of cytokines which reduces potential side-effects of a therapy using the composition according to the present invention. Moreover, the T cells activated by the method as described above show a reduced sensitivity to inactivating mechanisms in a patient, since the T cells activated according to the present invention are more differentiated and more efficiently activated.

For example, the methods according to the present invention can be used for obtaining a pharmaceutical composition containing a pharmaceutically effective amount of the T cell activating agent and/or the composition obtained according to the present invention, optionally in combination with a pharmaceutically acceptable carrier and/or diluent, preferably for the curative or prophylactic treatment of cancer, infections and autoimmune diseases. Furthermore, the pharmaceutical composition which may be obtained using the present invention may contain one or more other T cell activating agents which may act additively or synergistically with the T cell activating agent and/o composition according to the present invention. The pharmaceutical composition which may be obtained using the present invention may be applied by any conventional application route used in vaccination or cell therapy such as intravenous, intramuscular, intracutaneous, subcutaneous and/or intralymphatic administration, for example by infusion or injection.

The pharmaceutical composition which may be obtained using the present invention may be applied in a method for the treatment of a patient suffering from an impairment of the immune system which may be caused by disorders such as by cancer, infections, renal failure, autoimmune diseases and/or inherited immunodysfunctions comprising the step of administering the above-defined pharmaceutical composition in an amount sufficient to stimulate and/or to perform a specific immunoresponse in the patient.

Further embodiments of the present invention relate to methods for the preparation of activated dencritic cells and activated T cells, respectively. The method for the preparation of activated dendritic cells according to the present invention comprises the steps of
(a) preparing as an antigen a tumor cell infected with newcastle disease virus (NDV) *in vitro,*
(b) preparing monocytes from the patient or a relative thereof,
(c) developing dendritic cells from the monocytes by incubation *in vitro,*
(d) coincubating the obtained dendritic cells with the antigen obtained in step (a) *in vitro,*
wherein the virus is capable of improving the adhesion of the antigen to and the presentation of the antigen by the dendritic cells and of modulating the activation, maturation, stability and cosignalling of the dendritic cells, wherein steps (a) and (b) do not involve any surgical treatment of a human's or animal's body, and wherein the relative of the patient is a person or animal being related by blood and/or genetically via HLA-type with the patient.

The method for the prepration of activated T cells according to the present invention comprises the steps of
(i) preparing T cells from a patient or relative thereof,
(ii) treating the T cells with the T cell activating agent obtained by the method according to the present invention *in vitro*,
wherein step (i) does not involve any surgical treatment of the patient's or relative's body and wherein the relative of the patient is a person or animal being related by blood and/or genetically via HLA-type with the patient.

The present invention will be further illustrated by the following non-limiting example.

### EXAMPLE

### Adoptive cell therapy with allogeneic TCs which have been activated in vitro with NDV-modified DCs loaded with tumor cell material.

### Preparation of the antigen from the patient

The preparation of NDV-modified tumor cells comprises the following steps:
(1) Isolation of tumor material by surgical intervention
(2) Dissociation of tumor cell material into a suspension of single cells by mechanical and enzymatic means: four times incubation for 30 min at 37°C with collagenase (5 U/ml) and DNase (15 U/ml). Optionally, immunobead purification of tumor cells which reduces contaminating non-tumor cells.
(3) Cryoconservation of tumor cells
(4) Thawing of tumor cells and modification/infection with NDV: 20 to 100 hemagglutinating units of virus per 1x10⁷ cells.
(5) Inactivation of NDV-modified tumor cells by 4 to 5 cycles of shock freezing and thawing.

### Preparation of T cells and dentritic cells from a relative of the patient

(1) Taking bone marrow and/or blood from the relative
(2) Preparing monocytes from the bone marrow and/or blood and inducing maturation and differentiation into DCs by standard cultivation with interleukin-4 (IL-4), granulocyte macrophage colony stimulating factor (GM-CSF) and tumor necrosis factor (TNF) with and without NDV
   (i) isolation of cells from 150 ml blood
   (ii) cultivation of monocytes for one day in RPMI 1640 plus 2 mM glutamine, 100 U/ml penicillin, 100 µg/ml streptomycin and supplemented with 5% autologous, noninactivated plasma, 1000 U/ml IL-4 (from Promocell) and 1000 U/ml GM-CSF
   (iii) change of medium and cultivation for two days in the above medium (day 1)
   (iv) change of medium, addition of TNFα (from Promocell) at a final concentration of 10 ng/ml (day 4)
(3) Loading/Pulsing of DCs with virus-modified tumor cell lysate by adding the lysate to the DCs followed by incubation in cell culture medium
   (v) addition of DCs to tumor cells at a ratio of 1 part DCs to 3 parts dead tumor cells in 1 ml X-vivo medium, centrifugation at low speed [1000 revolutions per hour (rph)], incubation at 37°C for 4 h (day 5)
   (vi) control of antigen-loaded DCs using fluorescence-activated cell sorting (FACS) analysis
   (vii) cultivation of antigen-loaded DCs in RPMI + 5 % plasma + IL-4 + GM-CSF + TNFα for three days (until day 8)
(4) Isolation of TCs from bone marrow and/or blood by a method comprising an immunobead enrichment step (this may be carried out during the generation/loading phase of the TCs)
   (viii) isolation of TCs from 150 ml blood (erythroycte lysis, adherence, Pan T cell isolation kit)
   (ix) control of TCs using FACS analysis
(5) Expansion of TCs in cell culture
(6) Coincubation of TCs and DCs which have been pulsed with NDV-modified tumor lysate which comprises a short incubation in the presence of cytokines at low- or medium-dose in order to avoid induction of dependency of the TCs on these cytokines
   (x) addition of TCs to the antigen-loaded DCs in a ratio of 1 part DCs to 34 parts TCs and incubation in fresh RPMI supplemented with 5% plasma (but without cytokines) for three days (until day 11)
   (xi) change of medium and addition of IL-2 (6000 U/ml) on day 11 and cultivation for three days
   (xii) harvesting the activated TCs, resuspending the TCs in 10 ml medium I, filling the suspension in a syringe for intravenous injection, filtration; the filtrate is taken up in 400 µl medium I and injected subcutaneously.
(7) Analysis of antitumor activity of the activated TCs using ELISPOT T cells are coincubated (challenged) with antigen for 20 h. Thereafter, γ-interferon (IFN-γ) production is detected for each single T cell on a plate coated with anti-IFN-γ antibodies. Bound IFN-γ is detected in spots surrounding the T cells by means of ELISA stain.

### Therapy

### (1) Day 1 to 14

Immunosuppression of the patient with medium-dose or dose-intensified chemotherapy and/or radiotherapy and/or corticosteroids and cyclosporin, which is necessary in order to avoid the rejection of the allogeneic DC-NDV tumor-actived TCs of the patient's relative.

The patient is treated with 80 mg/m² taxol, 40 mg/m² epirubicin and 50 mg of hydrocortisol per day for two consecutive weeks. In addition, 30 Gray irradiations of a bone metastasis were carried out.

### (2) Day 15

Intravenous infusion of the allogeneic TCs which were activated by NDV-DC treatment

About 5x10⁸ T cells which have been activated by the above-described method are infused in a volume of 250 ml Ringer lactate solution.

### (3) Next cycle of treatment after a break of six weeks.

### Materials

Recombinant human (rHU) IL-4cc dissolved in phosphate buffered saline (PBS)/1% human serum albumin (HSA) (stock solution: 1x10⁵ U/ml, corresponding to 1x10⁵ µg/ml)

GM-CSF dissolved in PBS/1% bovine serum albumin (BSA) (stock solution: 1x10⁵ U/ml)

rHU TNFα dissolved in RPMI 1640/1% BSA (stock solution: 1 µg/ml) IL-2 dissolved in X-vivo (stock solution: 6x10⁵ U/ml, diluted 1:100), proleukin (from Chiron)

### Increased T cell activating properties of dendritic cells when pulsed with virus-treated antigen versus non-treated antigen

### Virus-treated versus non-treated antigen

MCF-7 cells were cultured in RPMI medium, supplemented with 10 % fetal calf serum (FCS). 1 x 10⁷ cells were washed in order to remove FCS and infected with 60 Hemaglutinating Units of Newcastle Disease Virus strain Ulster in RPMI medium by adding virus solution for 60 min at 37°C. Non-adsorbed virus were washed-off again before an incubation for 24 h at 37°C in RPMI/2% FCS was carried out.

Control cells were not infected with virus but otherwise treated in the same way as infected cells (i.e. incubation for 60 min in RPMI without FCS followed by incubation for 24 h in RPMI/2% FCS).

After the incubation was completed, infected (MCF-7-NDV) and control cells were lysed by three cycles of freeze-thawing. Protein content was estimated in both preparations.

### Preparation of dentritic cells

(1) Taking bone marrow from a breast cancer patient
(2) Preparing dendritic cells from the bone marrow as described above under item (2) of "preparation of T cells and dendritic cells from a relative of the patient".

### Loading/Pulsing dendritic cells with antigen

1 x 10⁶ dendritic cells were coincubated with 200 µg/ml lysed MCF-7-NDV or with 200 µg/ml lysed non-infected MCF-7-cells. This was carried out by washing dendritic cells and adding the washed cells to the corresonding antigenic protein solution.

### Activation of T cells with antigen-pulsed dendritic cells

Autologous T cells from the patient were prepared from bone marrow as described above under item (4) of "preparation of T cells and dendritic cells from a relative of the patient". Antigen-pulsed dendritic cells were added to the T cells in a ratio of one dendritic cell to five T cells. Incubation was carried out for 48 h.

### Determination of anti-tumor memory T cell response with ELISPOT

Activated T cells were determined on a single cell basis by their γ-interferon production using the ELISPOT assay. Bound γ-interferon is detected in spots surrounding the T cells by means of an ELISA stain as described above under item (7) of "preparation of T cells and dendritic cells from a relative of the patient".

### Results

415 spot forming cells in 2,5 x 10⁴ T cells were detected after simulation with MCF-7-NDV-pulsed dendritic cells. Only 190 spot forming cells were dected in 2,5 x 10⁴ T cells stimulated with non-infected MCF-7 cells. 150 spot forming cells were detected in 2,5 x 10⁴ T cells stimulated with non-pulsed dendritic cells. Less than 12 spots were observed in 2,5 x 10⁴ T cells not stimulated at all. 165 spots were counted when dendritic cells had been pulsed with a non-breast cancer cell line.

### Conclusion

The above results show that in the breast cancer patient the T cell stimulatory capacity of the dendritic cells which had been pulsed with virus-infected antigen was more than doubled in comparison to dendritic cells which had been pulsed with non-infected antigen, non-pulsed dendritic cells and dendritic cells which had been pulsed with a non-breast cancer cell line.

### Stability of increased stimulating properties of dendritic cells which have been pulsed with virus-infected tumor cells

Irradiated MCF-7 tumor cells used as antigen were infected for 30 min with NDV and stored overnight at 4°C without further incubation.

As a control, non-infected MCF-7 cells were used which were otherwise treated in the same way as NDV-infected cells. As a further control, peripheral blood leukocytes were used.

Dendritic cells were generated by incubation of monocytes from peripheral blood of a breast cancer patient with GM-CSF and interleukin-4 (IL-4) for 5 days using a standard protocoll (cf., for example, ref. 8.).

Dendritic cells were pulsed with infected, irradiated but non-lysed MCF-7 cells or control cells by coincubation at 37°C for 6 h in medium without cytokines. After 6 h TNF-α, IL-1, IL-6 and prostaglandin E2 were added to the cultures in order to support final differentiation of dendritic cells. Thereafter, incubation was continued for 40 h.

Pulsed dendritic cells were washed in order to remove cytokines. The washed cells were stored at 4°C for 6 h, followed by incubation for 90 h at 37°C in medium containing autologous serum but no cytokines. This procedure immitates storage of a vaccine at 4°C and then *in vivo* persistence of pulsed dendritic cells in the autologous patient after injection of the vaccine.

After 90 h the antigen-pulsed dendritic cells were used for short term stimulation (42 h) of autologous T cells purified from peripheral blood of the patient. The ratio of dendritic cells to T cells was from 1 to 10 to 1 to 100.

After 42 h of short term stimulation γ-interferon production (activation) in T cells was determined by the above-described ELISPOT method.

### Results

Dendritic cells, pulsed with virus-infected antigen (MCF-7 tumor cells), induced substantially more γ-interferon producing (i.e. activated) T cells than those dendritic cells which had been pulsed with control cells. Furthermore, the dendritic cells pulsed with virus-infected MCF-7 cells stimulated T cells more efficiently than virus-infected MCF-7 cells alone, non-infected MCF-7 cells alone, virus-pulsed dendritic cells or dendritic cells pulsed with peripheral blood leucocytes. Thus, the effect of virus enhancement of dendritic cell stimulatory activities was stable even after more than 90 hours of incubation without cytokines. Therefore, a T cell activating agent used as a vaccine containing these cells is capable of maintaining its *in vivo* T (memory) cell stimulating activity for at least this time period.

### References

1. Rosenberg SA, Lotze MT, Muul LM, Chang AE, Avis FP, Leitmann, Linehan WM, Robertson CN, Lee RE, Rubin JT, Seipp CA, Simpson RN, White DE: A progress report on the treatment of 157 patients with advanced cancer unsing lymphokine-activated killer cells and interleukin-2 or high-dose interleukin-2 alone. N Engl J Med 316. (15) 889 - 897, 1987
2. Rosenberg SA, Packard BS, Aeberseld PM, Solomon D, Topalian SL, Toy ST, simon P, Lotze MT, Vang JC, Seipp CA, Simpson C, carter C, Bork S, Schwarzentruber D, Wei JP, White DE: Use of tumorinfiltrating lymphocytes and interleukin-2 in the immunotherapy of patients with metastatic melanoma. New Engl J Med 319. 1676 - 1680, 1988
3. Fichtner K-P, Schirrmacher V, Griesbach A, Hull WE: In Vivo 1H-NMR microimaging with respiratory triggering for monitoring adoptive immuno-therapiy of metastatic mouse lymphoma. Magnetic Resonance In Medicine (MRM) 38. 440 - 455, 1997
4. Yamagishi H, Ueda Y, Oka T: A case report of immunotherapy on a patient with advanced gastric cancer by adoptive transfer of OK-432 reactive HLA-matched allogeneic lymphocytes. Cancer Immunol Immunother 46. 113 - 119, 1998
5. Schirrmacher V, Beckhove P, Krüger A, Rocha M, Umanski V, Fichtner K-P, Hull WE, Zangemeister-Wittke U, Griesbach A, Jurianz K, Hoegen Pv: Effective immune rejection of advanced metastasized cancer. Int J Oncol 6. 505 - 521, 1995
6. Cardoso AA, Seamon MJ, Afonso HM, Ghia P, Boussiotis VA, Freeman GJ, Gribben JG, Sallan SE, Nadler LM: Ex vivo generation of human anti-pre-B leukemia-specicic autologous cytolytic T cells. Blood 90. (2) 549 - 561, 1997
7. Tani M, Tanimura H, Yamaue H, Mizobata S, Iwahashi M, Tsunoda T, Noguchi K, Tamai M, Hotta T, Terasawa H, Arii K: Generation of CD4+ cytotoxic T-lymphocytes stimulated by immobilized anti-CD3 monoclonal antibody and interleukin-2 in cancer patients. Int J Cancer 60. 802 - 807, 1995
8. Nestle FO, Alijagic S, Gilliet M, Sun Y, Grabbe S, Dummer R, Burg G, Schadendorf D: Vaccination of melanom patients with peptide- or tumor lysate-pulsed dendritic cells. Nature Medicine 4. (3) 328 - 332, 1998
9. Höltl L, Rieser C, Papesh C, Ramoner R, Herold M, Klocker H, Radmayr C, Stenzl A, Bartsch G, Thumher M: Cellular and humoral immune responses in patients with metastatic renal cell carcinoma after vaccination with antigen pulsed dendritic cells. J Urol 161. 777 - 782, 1999
10. Hsu FJ, Benike C, Fagoni F, Liles TM, Czerwinski D, Taidi B, Engleman EG, Levy R: Vaccination of patients with B-cell lymphoma using autologous antigen-pulsed dendritic cells. Nature Medicine 2.52 - 58, 1996
11. Salgaller ML, Lodge PA, McLean JG, Tjoa BA, Loftus DJ, Ragde H, Kenny GM, Rogers M, Boynton AL, Murphy GP: Report of immune monitoring of prostate cancer patients undergoing T cell therapy using dendritic cells pulsed with HSA-A2-specific peptides from prostate-specific membrane antigen (PSMA). Prostate 35. 144 - 148, 1998
12. Bennet SRM: Induction of a CD8- cytotoxic T lymphocyte response by crosspriming requires cognate CD4-help. J Exp Med 186. 65 - 70, 1997
13. Peters JH, Gieseler R, Thiele B, Steinbach F: Dendritic cells: from ontogenetic orphans to myelomonocytic descendants. Immunology today 17. (6) 273 - 278,1996
14. Ahlert T and Schirrmacher V. Isolation of a human melanoma adapted Newcastle Disease Virus mutant with highly selektive replication patterns, canc res 1990;505962-8.
15. Ahlert T, Kaufmann M, Bastert G, Schirrmacher V. : Active Specific Immunotherapy (ASI) with virally modified autologous tumor cells in breast and ovarian cancer: factors influencing immunological vaccination success. J of Canc Res and Clin Oncol 116 / supplement. :80, 1990 (abstr)
16. Cassel WA, Murray DR: A ten-year follow-up on stage II malignang melanoma patients treated postsurgically with Neuwcastle disease virus oncolysate. Med Oncol & Tumor Pharmacother 9.169 - 171, 1992
17. Cassel WA, Garret RE: Newcastle Disease Virus as an antineoplastic agent. Cancer 66. (7) 1517 - 1523, 1965
18. Csatary LK, Eckhard S, Bukosza I, Czegledi F, Fenyvesi C, Gergely P, Bodey B, Csatary CM: Attenuated veterinary virus vaccine for the treatment of cancer. Cancer Detection and Prevention 17. 619 - 627, 1993
19. Schirrmacher V, Haas C, Bonifer R, Ahlert T, Gerhards R, Ertel C: Human tumor cell modification by virus infection: an efficient and safe way to produce cancer vaccine with pleiotropic immune stimulatory properties when using Newcastle disease virus. Gene therapy 6. 63 - 73, 1999
20. Schirrmacher V, Ahlert T, Pröbstle T, Steiner H-H, Herold-Mende C, Gerhards R, Hagmüller E: Immunisation with virus modified tumor cells. Seminars in oncology 25. (6) 677 - 696, 1998
21. Schirrmacher V, Hoegen von P, Heicappell R: Virus modified tumor cell vaccines for active specific immunotherapy of mecrometastases: Expansion and activation of tumor-specific T cells. Immunity to Cancer 2. 391 - 399, 1989
22. Schirrmacher V, Ahlert T, Heicappell R, Appelhans B, Hoegen von P: Successful application of non-oncogenic viruses for antimetastatic cancer immunotherapy. Cancer Res 5. 19 - 49, 1986
23. Ahlert T, Sauerbrei W, Bastert G, Ruhland S, Bartik B, Simiantonaki N, Schumacher J, Häcker B, Schuhmacher M, Schirrmacher V: Tumor-cell number and viabilitiy as quality and efficacy parameters of autologous virus-modified cancer vaccines in patients with breast or ovarian cancer - Errata. Journal of clinical oncology 15. (4) 2763, 1997
24. Schirrmacher V, Griesbach A, Zangemeister-Wittke U: y-irradiated viable tumor cells as whole-celle vaccines can stimulate in situ syngenelc antitumor cytotoxic T lymphocytes and delayed-type hypersensitivity reactivity whereas tumor cell lysates elicit only delayed-type hypersensitivity reactivity. Vaccine Res 3 - No.1. 31 - 48, 1994
25. Schirrmacher V, Hoegen von P: Importance of tumor cell membrane integrity and viability for cytotoxic T lymphocyte activation by cancer vaccines. Vaccine Res 2 - No. 3. 183 - 196, 1993
26. Ahlert T, Sauerbrei W, Bastert G, Ruhland S, Bartik B, Simiantonaki N, Schumacher J, Häcker B, Schumacher M, Schirrmacher V: Tumor cell number and viability as quality and efficacy parameters of autologous virus modified cancer vaccines. J Clin Oncol 15. 1354 - 1366, 1997
27. Jenne L et al., Immunobiology 200 (1999) 3-5, pp. 562
28. Raftery M et al., Immunobiology 200 (1999) 3-5, pp. 568

## Claims

1. A method for the preparation of a composition containing activated T cells which are capable of performing and stimulating a specific immune response in a patient, comprising the steps of
(i) preparing T cells from the patient or a relative thereof;
(ii) activating dendritic cells by a method comprising the steps of
(a) preparing as an antigen a tumor cell infected with newcastle disease virus (NDV) *in vitro,*
(b) preparing monocytes from the patient or a relative thereof,
(c) developing dendritic cells from the monocytes by incubation *in vitro,*
(d) coincubating the obtained dendritic cells with the antigen obtained in step (a) *in vitro,*
wherein the virus is capable of improving the adhesion of the antigen to and the presentation of the antigen by the dendritic cells and of modulating the activation, maturation, stability and cosignalling of the dendritic cells;
(iii) preparing a T cell activating agent comprising the dendritic cells of step (ii); and
(iv) activating the T cells of step (i) by treatment with the T cell activating agent of step (iii) *in vitro,*
wherein steps (i), (ii) (a), and (ii) (b) do not involve any surgical treatment of a human's or animal's body and wherein the relative of the patient is a person or animal being related by blood and/or genetically via HLA-type with the patient.

2. The method according to claim 1, wherein the treatment of the T cells comprises a short time incubation with the activated dendritic cells of not longer than 7 days.

3. The method according to claim 1 or 2, wherein the activated T cells are at least in part T memory cells.

4. The method according to any one of claims 1 to 3, wherein at least part of the T cells to be activated and/or at least part of the monocytes are prepared from the patient's or relative's bone marrow.

5. The method according to any one of claims 1 to 4, wherein the treatment of the T cells is carried out in a culture medium containing not more than 6000 U/ml of IL-2.

6. The method according to any one of claims 1 to 5, wherein the method further comprises the step of inactivating the antigen obtained in step (a) without the use of irradiation.

7. The method according to claim 6, wherein the antigen is inactivated by freeze-thawing or ultrasonification.

8. The method according to any one of claims 1 to 7, wherein the tumor cell is prepared from the patient.

9. The method according to any one of claims 1 to 8, wherein the tumor cell is further purified by immunobead techniques.

10. The method according to any one of claims 1 to 9, wherein the antigen is cryoconservated after its preparation and thawed before the coincubation with the dendritic cells in step (d).

11. The method according to any one of claims 1 to 10, wherein in step (c) the developing dendritic cells are also coincubated with the virus.

12. The method according to any one of claims 1 to 11, wherein the virus induces at least in part fusions between the antigen and the dendritic cells in step (d).

13. The method according to any one of claims 1 to 12, wherein the patient's immune system is significantly impaired.

14. The method according to claim 13, wherein the impairment of the immune system is caused by cancer, infections, renal failure, autoimmune diseases and/or inherited immunodysfunctions.

15. The method according to any one of claims 1 to 14, wherein the patient is a human.

16. The method according to any one of claims 1 to 15, wherein the T cell activating agent further contains one or more other T cell activating agents.

17. A method for the preparation of a T cell activating agent containing activated dendritic cells for the activation of T cells which perform and stimulate a specific immune response in a patient, wherein the dendritic cells are activated by the method comprising the steps of
(a) preparing as an antigen a tumor cell infected with newcastle disease virus (NDV) *in vitro,*
(b) preparing monocytes from the patient or a relative thereof,
(c) developing dendritic cells from the monocytes by incubation *in vitro,*
(d) coincubating the obtained dendritic cells with the antigen obtained in step (a) *in vitro,*
wherein the virus is capable of improving the adhesion of the antigen to and the presentation of the antigen by the dendritic cells and of modulating the activation, maturation, stability and cosignalling of the dendritic cells, wherein steps (a) and (b) do not involve any surgical treatment of a human's or animal's body, and wherein the relative of the patient is a person or animal being related by blood and/or genetically via HLA-type with the patient.

18. The method according to claim 17, wherein the method further comprises the step of inactivating the antigen obtained in step (a) without the use of irradiation.

19. The method according to claim 18, wherein the antigen is inactivated by freeze-thawing or ultrasonification.

20. The method according to any one of claims 17 to 19, wherein the tumor cell is prepared from the patient.

21. The method according to any one of claims 17 to 20, wherein the tumor cell is further purified by immunobead techniques.

22. The method according to any one of claims 17 to 21, wherein the antigen is cryoconservated after its preparation and thawed before the coincubation with the dendritic cells in step (d).

23. The method according to any one of claims 17 to 22, wherein in step (c) the developing dendritic cells are also coincubated with the virus.

24. The method according to any one of claims 17 to 23, wherein the virus induces at least in part fusions between the antigen and the dendritic cells in step (d).

25. The method according to any one of claims 17 to 24, wherein the patient's immune system is significantly impaired.

26. The method according to claim 25, wherein the impairment of the immune system is caused by cancer, infections, renal failure, autoimmune diseases and/or inherited immunodysfunctions.

27. The method according to any one of claims 17 to 26, wherein the patient is a human.

28. The method according to any one of claims 17 to 27, wherein the T cell activating agent further contains one or more other T cell activating agents.

29. A method for the preparation of activated dendritic cells comprising the steps of
(a) preparing as an antigen a tumor cell infected with newcastle disease virus (NDV) *in vitro,*
(b) preparing monocytes from the patient or a relative thereof,
(c) developing dendritic cells from the monocytes by incubation *in vitro,*
(d) coincubating the obtained dendritic cells with the antigen obtained in step (a) *in vitro,*
wherein the virus is capable of improving the adhesion of the antigen to and the presentation of the antigen by the dendritic cells and of modulating the activation, maturation, stability and cosignalling of the dendritic cells, wherein steps (a) and (b) do not involve any surgical treatment of a human's or animal's body, and wherein the relative of the patient is a person or animal being related by blood and/or genetically via HLA-type with the patient.

30. A method for the preparation of activated T cells comprising the steps of
(i) preparing T cells from a patient or relative thereof,
(ii) treating the T cells with the T cell activating agent obtained by the method according to any one of claims 17 to 28 *in vitro*,
wherein step (i) does not involve any surgical treatment of the patient's or relative's body and wherein the relative of the patient is a person or animal being related by blood and/or genetically via HLA-type with the patient.

## Patentansprüche

1. Verfahren zur Herstellung einer Zusammensetzung, enthaltend aktivierte T-Zellen, die befähigt sind, eine spezifische Immunantwort in einem Patienten durchzuführen und zu stimulieren, umfassend die Schritte
(i) des Herstellens von T-Zellen aus dem Patienten oder einem Verwandten von ihm,
(ii) des Aktivierens dendritischer Zellen durch ein Verfahren, umfassend die Schritte
(a) des Herstellens einer Tumorzelle, die *in vitro* mit Newcastle-Disease-Virus (NDV) infiziert wird, als ein Antigen,
(b) des Herstellens von Monozyten aus dem Patienten oder einem Verwandten von ihm,
(c) des Ausbildens dendritischer Zellen aus den Monozyten durch *in vitro* Inkubation,
(d) des Co-Inkubierens der erhaltenen dendritischen Zellen mit dem in Schritt (a) erhaltenen Antigen *in vitro,*
wobei das Virus befähigt ist, die Adhäsion des Antigens an und die Präsentation des Antigens durch die dendritischen Zellen zu verbessern und die Aktivierung, Reifung, Stabilität und das Co-Signalling der dendritischen Zellen zu modulieren,
(iii) des Herstellens eines T-Zell-aktivierenden Mittels, umfassend die dendritischen Zellen aus Schritt (ii), und
(iv) des Aktivierens der T-Zellen aus Schritt (i) durch *in vitro* Behandlung mit dem T-Zell-aktivierenden Mittel aus Schritt (iii),
wobei die Schritte (i), (ii) (a) und (ii) (b) keine chirurgische Behandlung eines menschlichen oder tierischen Körpers einschließen und wobei der Verwandte des Patienten eine Person oder ein Tier ist, die oder das mit dem Patienten blutsverwandt und/oder genetisch durch den HLA-Typ verwandt ist.

2. Verfahren nach Anspruch 1, wobei die Behandlung der T-Zellen eine kurzzeitige Inkubation mit den aktivierten dendritischen Zellen von nicht länger als 7 Tagen umfaßt.

3. Verfahren nach Anspruch 1 oder 2, wobei die aktivierten T-Zellen zumindest teilweise Gedächtnis-T-Zellen sind.

4. Verfahren nach einem der Ansprüche 1 bis 3, wobei mindestens ein Teil der zu aktivierenden T-Zellen und/oder mindestens ein Teil der Monozyten aus dem Knochenmark des Patienten oder des Verwandten hergestellt werden.

5. Verfahren nach einem der Ansprüche 1 bis 4, wobei die Behandlung der T-Zellen in einem Kulturmedium durchgeführt wird, das nicht mehr als 6000 U/ml IL-2 enthält.

6. Verfahren nach einem der Ansprüche 1 bis 5, wobei das Verfahren weiter den Schritt des Inaktivierens des in Schritt (a) erhaltenen Antigens ohne die Verwendung von Bestrahlung umfaßt.

7. Verfahren nach Anspruch 6, wobei das Antigen durch Einfrieren/Auftauen oder Behandlung mit Ultraschall inaktiviert wird.

8. Verfahren nach einem der Ansprüche 1 bis 7, wobei die Tumorzelle aus dem Patienten hergestellt wird.

9. Verfahren nach einem der Ansprüche 1 bis 8, wobei die Tumorzelle weiter durch Immunobead-Techniken gereinigt wird.

10. Verfahren nach einem der Ansprüche 1 bis 9, wobei das Antigen nach seiner Herstellung kryokonserviert wird und vor der Co-Inkubation mit den dendritischen Zellen in Schritt (d) aufgetaut wird.

11. Verfahren nach einem der Ansprüche 1 bis 10, wobei in Schritt (c) die sich ausbildenden dendritischen Zellen auch mit dem Virus co-inkubiert werden.

12. Verfahren nach einem der Ansprüche 1 bis 11, wobei das Virus mindestens teilweise Fusionen zwischen dem Antigen und den dendritischen Zellen in Schritt (d) hervorruft.

13. Verfahren nach einem der Ansprüche 1 bis 12, wobei das Immunsystem des Patienten signifikant beeinträchtigt ist.

14. Verfahren nach Anspruch 13, wobei die Beeinträchtigung des Immunsystems durch Krebs, Infektionen, Nierenversagen, Autoimmunerkrankungen und/oder vererbte Funktionsstörungen des Immunsystems verursacht wird.

15. Verfahren nach einem der Ansprüche 1 bis 14, wobei der Patient ein Mensch ist.

16. Verfahren nach einem der Ansprüche 1 bis 15, wobei das T-Zell-aktivierende Mittel weiter ein oder mehrere andere T-Zell-aktivierende Mittel enthält.

17. Verfahren zur Herstellung eines T-Zell-aktivierenden Mittels, enthaltend aktivierte dendritische Zellen für die Aktivierung von T-Zellen, die eine spezifische Immunantwort in einem Patienten durchführen und stimulieren, wobei die dendritischen Zellen durch das Verfahren aktiviert werden, umfassend die Schritte
(a) des Herstellens einer Tumorzelle, die *in vitro* mit Newcastle-Disease-Virus (NDV) infiziert wird, als ein Antigen,
(b) des Herstellens von Monozyten aus dem Patienten oder einem Verwandten von ihm,
(c) des Ausbildens dendritischer Zellen aus den Monozyten durch *in vitro* Inkubation,
(d) des Co-Inkubierens der erhaltenen dendritischen Zellen mit dem in Schritt (a) erhaltenen Antigen *in vitro,*
wobei das Virus befähigt ist, die Adhäsion des Antigens an und die Präsentation des Antigens durch die dendritischen Zellen zu verbessern und die Aktivierung, Reifung, Stabilität und das Co-Signalling der dendritischen Zellen zu modulieren, wobei die Schritte (a) und (b) keine chirurgische Behandlung eines menschlichen oder tierischen Körpers einschließen und wobei der Verwandte des Patienten eine Person oder ein Tier ist, die oder das mit dem Patienten blutsverwandt und/oder genetisch durch den HLA-Typ verwandt ist.

18. Verfahren nach Anspruch 17, wobei das Verfahren weiter den Schritt des Inaktivierens des in Schritt (a) erhaltenen Antigens ohne die Verwendung von Bestrahlung umfaßt.

19. Verfahren nach Anspruch 18, wobei das Antigen durch Einfrieren/Auftauen oder Behandlung mit Ultraschall inaktiviert wird.

20. Verfahren nach einem der Ansprüche 17 bis 19, wobei die Tumorzelle aus dem Patienten hergestellt wird.

21. Verfahren nach einem der Ansprüche 17 bis 20, wobei die Tumorzelle weiter durch Immunobead-Techniken gereinigt wird.

22. Verfahren nach einem der Ansprüche 17 bis 21, wobei das Antigen nach seiner Herstellung kryokonserviert wird und vor der Co-Inkubation mit den dendritischen Zellen in Schritt (d) aufgetaut wird.

23. Verfahren nach einem der Ansprüche 17 bis 22, wobei in Schritt (c) die sich ausbildenden dendritischen Zellen auch mit dem Virus co-inkubiert werden.

24. Verfahren nach einem der Ansprüche 17 bis 23, wobei das Virus mindestens teilweise Fusionen zwischen dem Antigen und den dendritischen Zellen in Schritt (d) induziert.

25. Verfahren nach einem der Ansprüche 17 bis 24, wobei das Immunsystem des Patienten signifikant beeinträchtigt ist.

26. Verfahren nach Anspruch 25, wobei die Beeinträchtigung des Immunsystems durch Krebs, Infektionen, Nierenversagen, Autoimmunerkrankungen und/oder vererbte Funktionsstörungen des Immunsystems verursacht wird.

27. Verfahren nach einem der Ansprüche 17 bis 26, wobei der Patient ein Mensch ist.

28. Verfahren nach einem der Ansprüche 17 bis 27, wobei das T-Zell-aktivierende Mittel weiter ein oder mehrere andere T-Zell-aktivierende Mittel enthält.

29. Verfahren zur Herstellung von aktivierten dendritischen Zellen, umfassend die Schritte
(a) des Herstellens einer Tumorzelle, die *in vitro* mit Newcastle-Disease-Virus (NDV) infiziert wird, als ein Antigen,
(b) des Herstellens von Monozyten aus dem Patienten oder einem Verwandten von ihm,
(c) des Ausbildens dendritischer Zellen aus den Monozyten durch *in vitro* Inkubation,
(d) des Co-Inkubierens der erhaltenen dendritischen Zellen mit dem in Schritt (a) erhaltenen Antigen *in vitro,*
wobei das Virus befähigt ist, die Adhäsion des Antigens an und die Präsentation des Antigens durch die dendritischen Zellen zu verbessern und die Aktivierung, Reifung, Stabilität und das Co-Signalling der dendritischen Zellen zu modulieren, wobei die Schritte (a) und (b) keine chirurgische Behandlung eines menschlichen oder tierischen Körpers einschließen und wobei der Verwandte des Patienten eine Person oder ein Tier ist, die oder das mit dem Patienten blutsverwandt und/oder genetisch durch den HLA-Typ verwandt ist.

30. Verfahren zur Herstellung von aktivierten T-Zellen, umfassend die Schritte
(i) des Herstellens von T-Zellen aus einem Patienten oder einem Verwandten von ihm,
(ii) des Behandelns der T-Zellen mit dem durch das Verfahren nach einem der Ansprüche 17 bis 28 erhaltene T-Zell-aktivierende Mittel *in vitro,*
wobei Schritt (i) keine chirurgische Behandlung des Körpers des Patienten oder des Verwandten einschließt und wobei der Verwandte des Patienten eine Person oder ein Tier ist, die oder das mit dem Patienten blutsverwandt und/oder genetisch durch den HLA-Typ verwandt ist.

## Revendications

1. Procédé de préparation d'une composition contenant des lymphocytes T activés qui sont capables de réaliser et de stimuler une réponse immunitaire spécifique chez un patient, comprenant les étapes consistant à
(i) préparer des lymphocytes T du patient ou d'un parent de celui-ci ;
(ii) activer des cellules dendritiques par un procédé comprenant les étapes consistant à :
(a) préparer comme antigène, une cellule tumorale infectée avec le virus de la maladie de Newcastle (NDV) *in vitro,*
(b) préparer des monocytes du patient ou d'un parent de celui-ci,
(c) développer des cellules dendritiques à partir des monocytes par incubation *in vitro,*
(d) co-incuber les cellules dendritiques obtenues avec l'antigène obtenu à l'étape (a) *in vitro,*
le virus étant capable d'améliorer l'adhérence de l'antigène aux cellules dendritiques et la présentation de l'antigène par les cellules dendritiques et de moduler l'activation, la maturation, la stabilité et le co-signalement des cellules dendritiques ;
(iii) préparer un agent activant les lymphocytes T comprenant les cellules dendritiques de l'étape (ii) ; et
(iv) activer les lymphocytes T de l'étape (I) par traitement avec l'agent d'activation des lymphocytes T de l'étape (iii) *in vitro,*
les étapes (i), (ii) (a) et (ii) (b) ne comprenant pas un traitement chirurgical d'un organisme humain ou animal et dans lequel le parent du patient est une personne ou un animal qui est apparenté hématologiquement et/ou génétiquement via le type HLA avec le patient.

2. Procédé selon la revendication 1, dans lequel le traitement des lymphocytes T comprend une courte durée d'incubation avec les cellules dendritiques activées qui ne dépasse pas 7 jours.

3. Procédé selon la revendication 1 ou 2, dans lequel les lymphocytes T activés sont au moins en partie des lymphocytes T mémoire.

4. Procédé selon l'une quelconque des revendications 1 à 3, dans lequel au moins une partie des lymphocytes T à activer et/ou au moins une partie des monocytes sont préparés à partir de la moelle osseuse du patient ou du parent.

5. Procédé selon l'une quelconque des revendications 1 à 4, dans lequel le traitement des lymphocytes T est réalisé dans un milieu de culture contenant moins de 6000 U/ml d'IL-2.

6. Procédé selon l'une quelconque des revendications 1 à 5, le procédé comprenant en outre l'étape d'inactivation de l'antigène obtenu à l'étape (a) sans utiliser d'irradiation.

7. Procédé selon la revendication 6, dans lequel l'antigène est inactivé par congélation - décongélation ou ultra-sonification.

8. Procédé selon l'une quelconque des revendications 1 à 7, dans lequel la cellule tumorale est préparée à partir du patient.

9. Procédé selon l'une quelconque des revendications 1 à 8, dans lequel la cellule tumorale est en outre purifiée par des techniques d'immunobilles.

10. Procédé selon l'une quelconque des revendications 1 à 9, dans lequel l'antigène est cryoconservé après sa préparation et décongelé avant la co-incubation avec les cellules dendritiques de l'étape (d).

11. Procédé selon l'une quelconque des revendications 1 à 10, dans lequel à l'étape (c), les cellules dendritiques en développement sont également co-incubées avec le virus.

12. Procédé selon l'une quelconque des revendications 1 à 11, dans lequel le virus induit au moins en partie, des fusions entre l'antigène et les cellules dendritiques de l'étape (d).

13. Procédé selon l'une quelconque des revendications 1 à 12, dans lequel le système immunitaire du patient est significativement perturbé.

14. Procédé selon la revendication 13, dans lequel la perturbation du système immunitaire est provoqué par le cancer, des infections, une insuffisance rénale, des maladies auto-immunes et/ou des dysfonctionnements immunitaires héréditaires.

15. Procédé selon l'une quelconque des revendications 1 à 14, dans lequel le patient est un être humain.

16. Procédé selon l'une quelconque des revendications 1 à 15, dans lequel l'agent activant les lymphocytes T contient en outre un ou plusieurs autres agents activant les lymphocytes T.

17. Procédé de préparation d'un agent activant les lymphocytes T contenant des cellules dendritiques activées pour l'activation des lymphocytes T qui réalisent et stimulent une réponse immunitaire spécifique chez un patient, les cellules dendritiques étant activées par un procédé comprenant les étapes consistant à
(a) préparer comme antigène, une cellule tumorale infectée avec le virus de la maladie de Newcastle (NDV) *in vitro,*
(b) préparer des monocytes du patient ou d'un parent de celui-ci,
(c) développer des cellules dendritiques à partir des monocytes par incubation *in vitro,*
(d) co-incuber les cellules dendritiques obtenues avec l'antigène obtenu à l'étape (a) *in vitro,*
le virus étant capable d'améliorer l'adhérence de l'antigène aux cellules dendritiques et la présentation de l'antigène par les cellules dendritiques et de moduler l'activation, la maturation, la stabilité et le co-signalement des cellules dendritiques ; les étapes (a) et (b) ne comprenant pas de traitement chirurgical d'un organisme humain ou animal, et dans lequel le parent du patient est une personne ou un animal qui est apparenté hématologiquement et/ou génétiquement via le type HLA avec le patient.

18. Procédé selon la revendication 17, dans lequel le procédé comprend en outre l'étape d'inactivation de l'antigène obtenu à l'étape (a) sans utiliser d'irradiation.

19. Procédé selon la revendication 18, dans lequel l'antigène est inactivé par congélation - décongélation ou ultra-sonification.

20. Procédé selon l'une quelconque des revendications 17 à 19, dans lequel la cellule tumorale est préparée à partir du patient.

21. Procédé selon l'une quelconque des revendications 17 à 20, dans lequel la cellule tumorale est en outre purifiée par des techniques d'immunobilles.

22. Procédé selon l'une quelconque des revendications 17 à 21, dans lequel l'antigène est cryoconservé après sa préparation et décongelé avant la co-incubation avec les cellules dendritiques de l'étape (d).

23. Procédé selon l'une quelconque des revendications 17 à 22, dans lequel à l'étape (c), les cellules dendritiques en développement sont également co-incubées avec le virus.

24. Procédé selon l'une quelconque des revendications 17 à 23, dans lequel le virus induit au moins en partie, des fusions entre l'antigène et les cellules dendritiques de l'étape (d).

25. Procédé selon l'une quelconque des revendications 17 à 24, dans lequel le système immunitaire du patient est significativement perturbé.

26. Procédé selon la revendication 25, dans lequel la perturbation du système immunitaire est provoquée par le cancer, des infections, une insuffisance rénale, des maladies auto-immunes et/ou des dysfonctionnements immunitaires héréditaires.

27. Procédé selon l'une quelconque des revendications 17 à 26, dans lequel le patient est un être humain.

28. Procédé selon l'une quelconque des revendications 17 à 27, dans lequel l'agent activant les lymphocytes T contient en outre un ou plusieurs autres agents activant les lymphocytes T.

29. Procédé de préparation de cellules dendritiques activées, comprenant les étapes consistant à
(a) préparer comme antigène, une cellule tumorale infectée avec le virus de la maladie de Newcastle (NDV) *in vitro,*
(b) préparer des monocytes du patient ou d'un parent de celui-ci,
(c) développer des cellules dendritiques à partir des monocytes par incubation *in vitro,*
(d) co-incuber les cellules dendritiques obtenues avec l'antigène obtenu à l'étape (a) *in vitro,*
le virus étant capable d'améliorer l'adhérence de l'antigène aux cellules dendritiques et la présentation de l'antigène par les cellules dendritiques et de moduler l'activation, la maturation, la stabilité et le co-signalement des cellules dendritiques ; les étapes (a) et (b) ne comprenant pas de traitement chirurgical d'un organisme humain ou animal, et dans lequel le parent du patient est une personne ou un animal qui est apparenté hématologiquement et/ou génétiquement via le type HLA avec le patient.

30. Procédé de préparation de lymphocytes T activés comprenant les étapes consistant à
(i) préparer des lymphocytes T d'un patient ou d'un parent de celui-ci,
(ii) traiter les lymphocytes T avec l'agent activant des lymphocytes T obtenu par le procédé selon l'une quelconque des revendications 17 à 28 *in vitro,*
dans lequel l'étape (i) n'implique pas de traitement chirurgical de l'organisme du patient ou du parent et dans lequel le parent du patient est une personne ou un animal qui est apparenté hématologiquement et/ou génétiquement via le type HLA avec le patient.
